Europäisches Patentamt

(19) European Patent Office

Office européen des brevets

(11) Publication number: **0 181 107 B1**

(12) **EUROPEAN PATENT SPECIFICATION**

(45) Date of publication of patent specification: **05.02.92**

(51) Int. Cl.5: **C12N 15/43**, G01N 33/53, G01N 33/68

(21) Application number: **85307497.9**

(22) Date of filing: **17.10.85**

(54) **Production of human T-cell leukemia (lymphotropic) retrovirus (htlv-1) envelope protein fragments in bacteria and use in seroepidemiological survey of human lymphoid malignancies.**

(30) Priority: **26.10.84 US 664972**

(43) Date of publication of application:
**14.05.86 Bulletin 86/20**

(45) Publication of the grant of the patent:
**05.02.92 Bulletin 92/06**

(84) Designated Contracting States:
**AT BE CH DE FR GB IT LI LU NL SE**

(56) References cited:
**EP-A- 0 113 078**

**Proceedings of the National Academy of Sciences of the United States of America, vol. 80, n 6, March 1983, Baltimore, USA. V. Manzari et al. "Human T-cell leukemia-lymphoma virus (HTLV) ; cloning of an integrated defective provirus and flanking cellular sequences",pages 1574-1578**

**Virology, vol. 136 (1984), pages 338-347**

**Science, vol. 24 (11.5.1984), pages 607-610**

(73) Proprietor: **THE UNITED STATES OF AMERICA as represented by the Secretary, United States Department of Commerce National Technical Information Service, Office of Government Inventions and Patents, 5285 Port Royal Road Springfield, Virginia 22161(US)**

(72) Inventor: **Papas, Takis S.**
**10700 Tulip Lane**
**Potomac Maryland 20859(US)**
Inventor: **Wong-Staal, Flossie**
**8418 Harker Drive**
**Potomac Maryland 20854(US)**
Inventor: **Samuel, Kenneth**
**8136 15th Avenue, Apartment 103**
**Hyattsville Maryland 20783(US)**
Inventor: **Lautenberger, James A.**
**4492 Willowtree Drive**
**Middletown Maryland 21769(US)**

Science, vol. 225 (27.7.1984), pages 421-424

P.N.A.S. (USA), 1983, vol. 80, pages 3618-3622

P.N.S.A. (USA), 1984, vol. 81, pages 3856-3860

P.N.S.A. (USA), 1984, vol. 81, pages 6202-6206

(74) Representative: **Jump, Timothy John Simon et al**
**F.J. Cleveland and Company 40-43 Chancery Lane**
**London WC2A 1JQ(GB)**

## Description

Two regions of the gene for the HTLV-I envelope were expressed in Escherichia coli by use of the vector pJLA16. One corresponds to the carboxyterminal region of the major envelope protein p46 and the other corresponds to the transmembrane protein p21E. Reactivity of the expressed protein with human sera was tested by Western blot procedure. Each of the sera tested that had been shown to contain anti-HTLV-I or anti-HTLV-II antibodies by ELISA assay recognized the bacterially synthesized envelope proteins. There was no reaction detected when control sera were tested. This system is useful for large scale seroepidemiological surveys for this and related human retroviruses.

It has been found, and is the subject of this invention, that where the HTLV envelope protein is isolated into fragments such as the 400 base pair (bp) and 300 bp, these fragments placed in a common vector and in bacteria can be utilized in competition testing where the protein here is used as the antigen and mixed with sera of patients to show that said competition analysis will be almost 100% positive for patients of HTLV-I.

Human T-cell leukemia virus subgroup I (HTLV-I) is a retrovirus causatively linked to certain adult lymphoid malignancies, notably adult T-cell leukemia-lymphoma (ATL). Two other isolates (HTLV-II), including one from a patient with T-cell hairy cell leukemia, are clearly related to HTLV-I but significantly differ in antigen assays and in their genomes. A third subgroup of HTLV (HTLV-III) has recently been described (Popovic, et al, Science, 224:497, 1984; Gallo et al, ibid., p. 500; and Sarngadharan et al, ibid., p. 506) that is associated with acquired immunodeficiency syndrome (AIDS).

Antibodies that react with HTLV-I proteins have been found in the sera of ATL patients. These antibodies recognize both the gag core antigens and the envelope proteins of the virus. Viral core proteins were readily purified, sequenced, and extensively used in immunoassays; however, progress with the more important viral envelope proteins was slower. A limiting factor, therefore, in the studies of the immune response to these viruses has been the difficulty in isolating the viral envelope proteins in pure form and in quantity. As an alternative approach, the present invention expresses the virus envelope protein in a bacterial vector. This procedure has the advantage that only a single viral product as defined by the structure of the input DNA is made by the bacteria. HTLV-I was suitable for such an approach since the integrated proviral DNA has been cloned [Seiki et al, Proc. Natl. Acad. Sci. USA, 79:6899 (1982) and Manzari et al, Proc. Natl. Acad. Sci. USA, 80:1574 (1983)] and sequenced [Seiki et al, Proc. Natl. Acad. Sci. USA, 80:3618 (1983)]. The HTLV-I envelope is expressed by placing it into the pJLA16 derivative [Lautenberger et al, Gene Anal. Techniques, 1:63-66 (1984)] of plasmid pJL6 [Lautenberger et al, Gene, 23:75 (1983)]. This plasmid contains the 13 amino-terminal codons of the bacteriophage lambda cII gene placed under the transcriptional control of the well-regulated phage lambda $p_L$ promoter. This plasmid is known and has been successfully used to express sequences from myc, myb, and ras oncogenes [Lautenberger et al, Gene, 23:75 (1983) and Lautenberger et al, in Gene Amplification and Analysis, Volume 3, Expression of Cloned Genes in Prokaryotic and Eukaryotic Cells, Papas et al (eds), Elsevier, New York/Amsterdam, pp. 147-174].

Initial attempts to express the entire HTLV-I envelope were unsuccessful, possibly because this protein can interact with the bacterial cell membrane in much a way as to be toxic to the cell.

Kiyokawa et al in 81 Proc. Natl, Acad of Sci, USA 6202-6206 October 1984 discloses a method of producing HTLV-I retroviral envelope proteins comprising the steps of:-
   (1) Isolating the envelope gene of a human lymphotrophic retrovirus;
   (2) Cleaving the envelope gene to provide gene fragments coding respectively for specific regions of the envelope;
   (3) Inserting the fragments formed in step (2) into vectors;
   (4) Transferring the vectors formed in step (3) into prokaryote hosts; and
   (5) Isolating the protein from lysated host cells.

The present invention is characterised by a method of producing HTLV-I retroviral envelope proteins comprising the steps of:-
   (1) Isolating the envelope gene of a human lymphotrophic retrovirus HTLV-I,
   (2) Cleaving the envelope gene to provide gene fragment coding respectively for specific regions of the envelope;
   (3) Inserting a fragment formed in step (2) into a vector,
   (4) Transferring the vector formed in step (3) into a prokaryote host; and lysing the host cells; and
   (5) Isolating said HTLV-I retrovirus envelope protein from the lysated host cells;
   characterised in that the cleaved fragment of step (2) is at least one gene fragment coding for a polypeptide containing antigenic sites reactive with human antibodies directed against HTLV-I or HTLV-II or related human retroviruses, said fragment being selected from a 321 base pair Xho1-Bam H2 envelope gene fragment and a 397 base pair Bam H1-Xho1 envelope gene fragment.

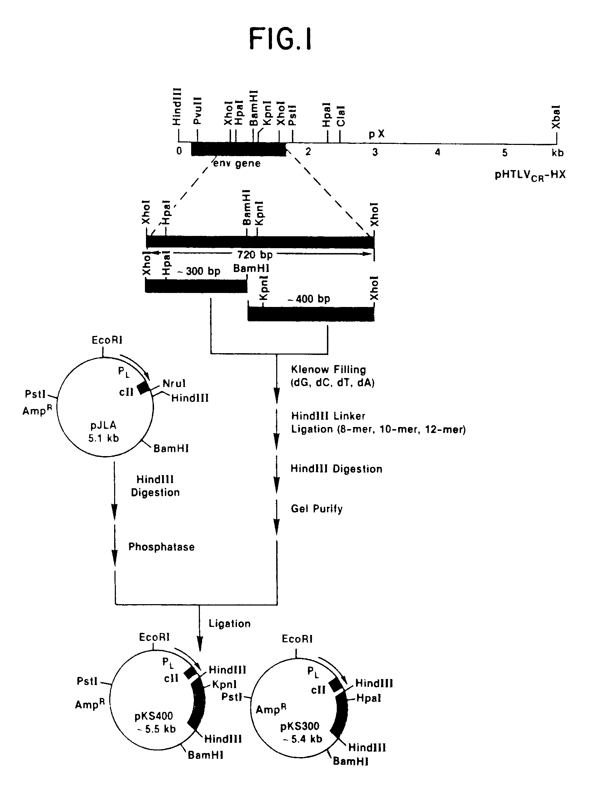

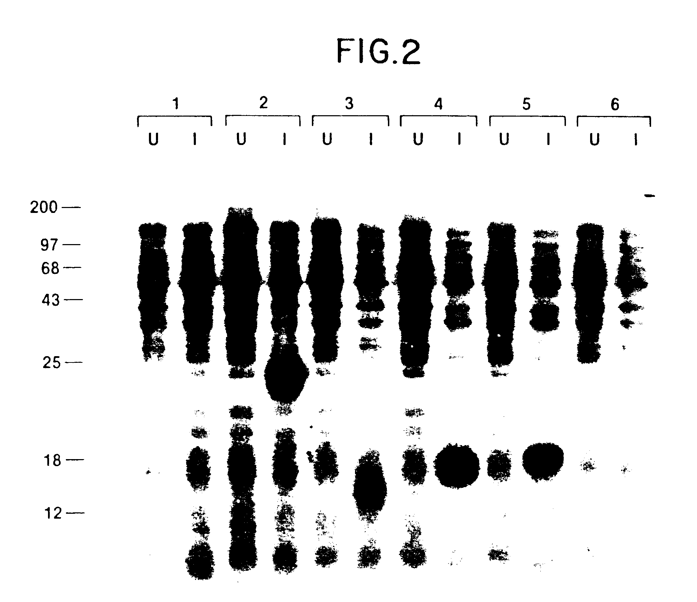

Thus in one embodiment individual fragments coding for specific regions of the envelope were inserted into pJLA6 by use of polynucleotide linkers (Fig. 1). Such plasmids were introduced into E. coli MZ1, a strain that contains a partial lambda prophage bearing the mutant cI857 temperature-sensitive repressor. At 32°C the repressor is active and $p_L$ promoter on the plasmid is repressed. At 42°C the repressor is inactive and the $p_L$ promoter is induced, allowing high level expression of genes under its transcriptional control. When lysogens carrying either of the two plasmids containing different portions of the HTLV-I envelope gene (cf. ante) were grown at 32°C and induced by shifting the temperature to 42°C, prominent bands were observed that were not found in uninduced cells or in induced cells containing the pJL6 vector alone (Fig. 2). These proteins were readily observed both in gels of radiolabeled bacterial extracts and gels stained for total protein. Based on DNA sequence data of the envelope gene fragments utilized in this study, the calculated molecular sizes of the pKS300 and pKS400 proteins are 12.84 Kd and 15.88 Kd, respectively. These sizes include the 1.56 Kd coding sequence contributed by the amino terminal codons of the lambda cII gene. The observed molecular weights of both proteins on SDS-polyacrylamide gels are consistent with those calculated for a 321 base pair (pKS300 insert) and 397 base pair (pKS400 insert) coding sequences or polypeptide sequences.

Statement of Deposit

Before the filing date of this application there were deposited in the American Type Culture Collection (ATCC) envelope gene fragments pKS300 and pKS400 respectively under the numbers ATCC No. 39902 and ATCC No. 39903. This depository assures permanence of the deposit and availability to the public upon the issuance of a patent related directly to this patent application.

Description of the Drawings

Figure 1 is the construction of plasmids pKS300 and pKS400.
Figure 2 is the expression of the HTLV-I envelope gene in E. coli.
Figure 3 is the recognition of bacterial synthesized HTLV-I envelope protein by antibodies in human serum.

Material Information Disclosure

Lautenberger et al, Gene Anal. Techniques, 1:63-66 (1984).
Lautenberger et al, Gene, 23:75 (1983).
Lautenberger et al, Science, 221:858 (1983).
Lautenberger et al, in Gene Amplification and Analysis, Vol. 3, Expression of Cloned Genes in Prokaryotic and Eukaryotic Cells, Papas et al (eds.), New York/Amsterdam: Elsevier, pp. 147-174.

In the invention the HTLV-I env gene codes for a glycoprotein (gp61) of molecular weight 61,000 that is cleaved into the molecular weight 46,000 exterior glycoprotein (gp46) and the molecular weight 21,000 trans membrane protein (gp21E). The precise site of proteolytic cleavage has been determined by locating radiolabeled valine residues with respect to the amino terminal end of gp21. The cleavage of the env gene precursor is adjacent to the residues Arg-Arg that also occur next to the proteolytic cleavage sites in the bovine leukemia virus (BLV) and mouse mammary tumor virus (MMTV) env precursor. Since the BamHI site that separates the inserted fragments is close to the region coding for proteolytic cleavage site that separates gp46 from p21E, the protein from pKS300 contains sequences corresponding to the carboxy-terminal portion of gp46 and the protein from pKS400 predominantly consists of sequences from p21E. See also preparation in Example 1.

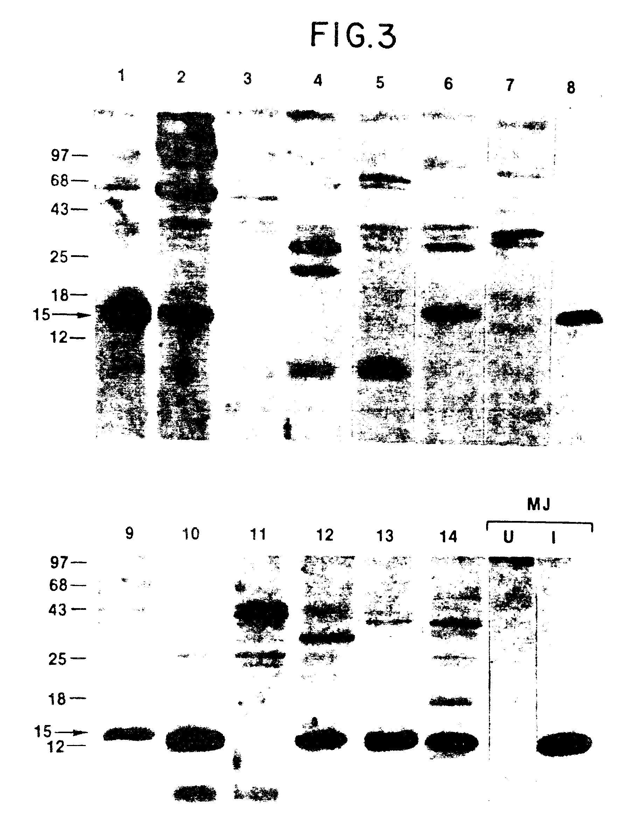

Sera from many patients with HTLV-I associated ATL and certain other lymphoid malignancies contain antibodies to proteins that have been shown to be the product of the viral env gene. In order to see if such antibodies can recognize a bacterially synthesize envelope product, a lysate of induced MZ1[pKS400] cells containing this protein was fractionated by SDS-polyacrylamide gel electrophoresis and transferred to nitrocellulose sheets by electrophoretic (Western) blotting. Strips containing the transferred proteins were reacted with diluted human serum and antigen-antibody complexes formed were detected by incubation of the strips with $^{125}$I-labeled Staphlococcus aureus protein A followed by autoradiography. As shown in Fig. 3, prominent bands corresponding to reaction of antibody to the 15 Kd bacterial envelope produce could readily be observed when the serum used was from patients with HTLV-I associated ATL or from HTLV-I antigen (+) individuals. No such reactions were observed with sera from healthy control individuals. This procedure was used to screen a group of 28 coded sera. Antibodies that recognized the bacterially synthesized HTLV-I envelope protein sequences were found in all sera that had been shown to have anti-HTLV-I antibodies by ELISA assay using disrupted virions as antigen (Table 1). Thus, a method is formulated for serologically testing for the presence in human sera of antibodies directed against HTLV-I or HTLV-II. None of the normal control sera were found to have reacting antibodies. Antibodies from a patient (Mo) with a hairy cell leukemia, whose disease is associated with HTLV-II, strongly reacted to the protein coded for in pKS400 indicating that there is a high degree of relatedness between the p21E region of HTLV-I and HTLV-II.

Since the bacterially synthesized HTLV-I env protein was recognized by antibodies present in sera from AIDS patients, it was also of interest to show that this assay can be utilized to screen for a more distantly related subgroup, namely, HTLV-III (the virus associated with AIDS). Therefore, a number of sera samples of AIDS patients, some of which were also sero-positive for HTLV-I, were examined.

## TABLE 1

### Presence of Antibodies Recognizing Bacterially Synthesized HTLV-I Envelope in Human Sera

| Status | HTLV-I or HTLV-II +/- (by ELISA) | Number Tested | Number Positive |
|---|---|---|---|
| Clinically normal heterosexual | + | 2 | 2/2 |
| | - | 8 | 0/8 |
| Clinically normal homosexual | - | 5 | 0/5 |
| AIDS patients | + | 2 | 2/2 |
| | - | 2 | 0/2 |
| ATL patients | + | 5 | 5/5 |
| Mycosis fungoides patient | + | 1 | 1/1 |
| Hairy cell leukemia patient Mo (HTLV-I + patient) | + | 1 | 1/1 |
| Lymphadenopathy syndrome patients | - | 2 | 0/2 |

The sera from all positive AIDS which reacted with HTLV-I in ELISA contained antibodies that recognized the bacterial synthesized HTLV-I env protein. None of the sera from AIDS patients that were HTLV-I negative contained antibodies that reacted with the bacterial protein. Since antibodies that react with HTLV-III proteins can be found in the serum of greater than 90% of AIDS patients, this result indicates that there is little or no cross reaction between the carboxy-terminal portion of the envelope proteins of HTLV-I and HTLV-III.

The results presented here demonstrate the importance of using bacterially synthesized proteins to study the properties of antibodies in human serum. Since the structure of the genes for such proteins can be controlled by recombinant DNA techniques, the antigens produced by these methods have a defined structure.

Example 1

Construction of plasmids pKS300 and pKS400. Plasmid pHTLV-I HX-CR was obtained by subcloning the 5.7 kb Hind III-XbaI fragment of lambda CR1 [Manzari et al, Proc. Natl. Acad. Sci. USA, 79:6899 (1982)] that contained envelope, pX, and LTR sequences. Lambda CR1 contained integrated HTLV-I proviral DNA from mycosis fungoides patient CR. pHTLV-I HX-CR DNA was digested XhoI and BamHI and the 300 bp and 400 bp fragments containing the env sequences were isolated from an agarose gel. The termini of these fragments were converted to blunt ends by the action of Klenow fragment E. coli DNA polymerase I and Hind III linkers were attached. Excess linkers were removed by digestion with Hind III and reisolation of the fragments from agarose gels. The pJLA16 [Lautenberger et al, Gene Anal. Techniques, 1:63-66 (1984)] vector DNA was cleaved with HindIII and the ends were dephosphorylated by the action of calf intestinal phosphatase. The dephosphorylated vector DNA was ligated to the fragment DNAs and introduced into DC646 cells by transformation using ampicillin selection. Plasmids containing inserts were identified by hybridization of colonies transferred to nitrocellulose with radiolabelled fragment produced by nick-translation fragment DNA using [$\alpha$-$^{32}$P]dCTP. For protein expression experiments, the plasmids were transferred into a prokaryote host such as by transferring into E. coli (strain MZ1) provided by M. Zuber and D. Court. Recombinant DNA procedures were as described by Maniatis et al, Molecular Cloning: A Laboratory Manual, Cold Spring Halbor Laboratory, Cold Spring Harbor, NY.

Example 2

Expression of the HTLV-I envelope gene in E. coli.

(a) Radiolabeling of bacterial cell proteins. E. coli MZ1 cells were grown at 32°C, induced by shifting the temperature to 41°C, labeled with [$^{35}$S]-cysteine and lysed. Proteins were resolved by sodium dodecyl sulfate-polyacrylamide gel electrophoresis (SDS-PAGE) and visualized by autoradiography.
(b) Uninduced (U) and induced (I) cell extracts of expression plasmid vectors - Lane 1, pJL6 vector without insert; Lane 2, pJLcII ras; Lane 3, pKS300; Lane 4, pKS400.1; Lane 5, pKS400.2; Lane 6, 400 bp fragment in wrong orientation. Confer Fig. 2.

Example 3

Recognition of bacterial synthesized HTLV-I envelope protein by antibodies in human serum.

MZ1 [pKS400] cells were grown at 32°C, induced at 42°C, and lysed in the presence of 1% SDS-0.1% beta-mercaptoethanol. Protein in the extracts were resolved by SDS-PAGE and electrophoretically transferred to nitrocellulose paper by the "Western blot" procedure. After transfer, filters were air dried and soaked in TBS-NDM (50 mM Tris-HCl, pH 7.5, 500 mM NaCl, 3% Nonfat Dry Milk). The filters were incubated overnight at room temperature in TBS-NDM plus 1/77 volume human serum as indicated below. Filters were then washed with TBS-NDM for 30 min and then incubated with 10$^5$ cpm [$^{125}$I]-proteinA (NEN). The filter was then washed with TBS-NDM and finally with TBS. The filters were air dried and protein bands reacting with antibody were visualized by autoradiography. The sera used were: (1) American ATL patient; (2) T-cell hairy cell leukemia pateint Mo (Ref. 4); (3) Healthy normal; (4) Health normal; (5) Healthy normal; (6) Healthy relative of ATL patient; (7) Healthy normal; (8) Japanese ATL patient; (9) AIDS patients found to be HTLV-II (+) by ELISA (disrupted virus antigen); (10) AIDS patient found to be HTLV-I (+) by ELISA (disrupted virus antigen); (11) Healthy normal; (12) American ATL patient; (13) Mycosis fungoides patient; (14) Healthy normal found to be HTLV-I (+) by ELISA (disrupted virus antigen). Uninduced and induced extracts pKS400.2 reacted with patients MJ serum (HTLV-I positive by ELISA).

**Claims**

1.   A method of producing HTLV-I retroviral envelope proteins comprising the steps of:-
    (1) Isolating the envelope gene of a human lymphotrophic retrovirus HTLV-I,
    (2) Cleaving the envelope gene to provide gene fragments coding respectively for specific regions of the envelope;
    (3) Inserting a fragment formed in step (2) into a vector,
    (4) Transferring the vector formed in step (3) into a prokaryote host; and lysing the host cells; and
    (5) Isolating said HTLV-I retrovirus envelope protein from the lysated host cells;

EP 0 181 107 B1

characterised in that the cleaved fragment of step (2) is at least one gene fragment coding for a polypeptide containing antigenic sites reactive with human antibodies directed against HTLV-I or HTLV-II or related human retroviruses, said fragment being selected from a ~ 321 base pair xho1-Bam H2 envelope gene fragment and a ~ 397 base pair Bam H1-Xho1 envelope gene fragment.

2. A method of claim 1 characterised in that the fragments of step (3) are inserted into the vector such that a portion of the lambda cII gene lies upstream to the inserted fragment.

3. A method of claim 1 or claim 2 characterised in that the envelope gene is cleaved into two fragments; one that codes for a glycoprotein and the other which codes for a transmembrane protein.

4. A method of any of claims 1 to 3 characterised in that the vector of step (3) is a plasmid.

5. A method of any preceding claim characterised in that the host cell is E. coli.

6. A method of claim 5 characterised in that the E. coli. is E. coli MZ1 which bears a mutant C1 857 temperature-sensitive repressor.

7. A method of any preceding claim characterised in that the vector of step (3) is a pJLA16 plasmid.

8. A method of claim 7 characterised in that the vector insert is pKS400, which is deposited under ATCC No. 39903.

9. A method of claim 7 characterised in that the vector inserted is pKS300, which is deposited under ATCC No. 39902.

10. A composition comprising peptides produced by the method of claim 1 on a solid support or carrier.

11. A method of detecting antibodies to HTLV-I, comprising contacting a composition of claim 10 with sera suspected of containing antibodies to HTLV-I.

12. A method of claim 11 characterised in that the peptides are attached to a solid support or carrier.

13. A method of claim 12 characterised in that the peptides are tested using an ELISA test.

14. A method of expressing in a bacterial vector a DNA fragment from the envelope coding region of HTLV-I whereby the polypeptide represented by said fragment can be used to detect antibody in human sera specifically directed against HTLV-I or HTLV-II characterised in that the DNA fragment is selected from a Bam H1-Xho1 fragment of ~ 397 base pairs in length which codes for a 15.88 Kd polypeptide sequence and a Xho1-Bam H1 fragment of ~ 321 base pairs which codes for a 12.84 Kd polypeptide sequence.

15. A method of serologically testing for the presence of human sera of antibodies directed against HTLV-I or HTLV-II and related human retroviruses, which comprises utilizing the product of claim 14.

16. A method according to either of claims 14 or 15 characterised in that an individual envelope gene fragment of HTLV-I is inserted into plasmid vector pJLA16 using a polynucleotide linker, said vector is introduced into E. coli. , and said E. coli. is grown at 32°C and induced at 42°C in a temperature sensitive reaction.

17. A method according to claim 16 characterised in that the E. coli. is E. coli. MZ1.

18. The ~ 321 base pair Xho1-Bam H1 envelope gene fragment of HTLV-I which codes for a polypeptide containing antigenic sites reactive with human antibodies directed against HTLV-I or HTLV-II or related human retroviruses.

19. The ~ 397 base pair Bam H1-Xho1 envelope gene fragment of HTLV-I which codes for a polypeptide

7

containing antigenic sites reactive with human antibodies directed against HTLV-I or HTLV-II or related human retroviruses.

20. An envelope antigen produced in E. coli carrying vector pJLA16 into which has been inserted an envelope gene fragment according to either of claims 18 or 19 in an amount which is suitable for detecting antibody in human sera specifically directed against HTLV-I.

**Revendications**

1. Procédé pour produire des protéines de l'enveloppe du rétrovirus HTLV-I comprenant :
   (1) l'isolement du gène de l'enveloppe d'un rétrovirus lymphotropique humain HTLV-I,
   (2) le clivage du gène de l'enveloppe pour obtenir des fragments de gène codant respectivement pour des régions spécifiques de l'enveloppe,
   (3) l'insertion d'un fragment obtenu à l'étape (2) dans un vecteur,
   (4) le transfert du vecteur obtenu à l'étape (3) dans un hôte procaryote ; et la lyse des cellules hôte ; et
   (5) l'isolement de ladite protéine de l'enveloppe du rétrovirus HTLV-I à partir des cellules hôte lysées,
   **caractérisé en ce que**
   le fragment clivé selon l'étape (2) est au moins un fragment de gène codant pour un polypeptide contenant des sites antigéniques présentant une réactivité vis-à-vis d'anticorps humains dirigés contre HTLV-I ou HTLV-II ou des rétrovirus humains apparentés, ledit fragment étant choisi parmi le fragment Xho1-Bam H2 du gène de l'enveloppe présentant ~ 321 paires de bases et le fragment Bam H1-Xho1 du gène de l'enveloppe présentant ~ 397 paires de bases.

2. Procédé selon la revendication 1, caractérisé en ce que les fragments de l'étape (3) sont introduits dans le vecteur de manière à ce qu'une partie du gène lambda cII soit située en amont du fragment inséré.

3. Procédé selon la revendication 1 ou la revendication 2, caractérisé en ce que le gène de l'enveloppe est clivé en deux fragments, l'un codant pour une glycoprotéine et l'autre codant pour une protéine transmembranaire.

4. Procédé selon l'une quelconque des revendications 1 à 3, caractérisé en ce que le vecteur de l'étape (3) est un plasmide.

5. Procédé selon l'une quelconque des revendications précédentes, caractérisé en ce que la cellule hôte est E. coli.

6. Procédé selon la revendication 5, caractérisé en ce que E. coli est E.coli MZ1 qui porte un répresseur mutant c1 857 sensible à la température.

7. Procédé selon l'une quelconque des revendications précédentes, caractérisé en ce que le vecteur de l'étape (3) est un plasmide pJLA 16.

8. Procédé selon la revendication 7, caractérisé en ce que le segment d'insertion du vecteur est le pKS 400, qui a été déposé auprès de l'ATCC sous le n° 39903.

9. Procédé selon la revendication 7, caractérisé en ce que le segment d'insertion du vecteur est le pKS 300, qui a été déposé auprès de l'ATCC sous le n° 39902.

10. Composition comprenant des peptides produits selon le procédé de la revendication 1 sur un support solide ou un porteur.

11. Procédé pour détecter des anticorps anti-HTLV-I comprenant la mise en contact d'une composition de la revendication 10 avec les sérums supposés contenir les anticorps anti-HTLV-I.

12. Procédé selon la revendication 11, caractérisé en ce que les peptides sont fixés sur un support solide ou sur un porteur.

**13.** Procédé selon la revendication 12, caractérisé en ce que les peptides sont testés selon un test ELISA.

**14.** Procédé pour l'expression, dans un vecteur bactérien, d'un fragment d'ADN de la région codant pour l'enveloppe de HTLV-I, selon lequel le polypeptide représenté par ledit fragment peut être utilisé pour détecter un anticorps dans des sérums humains spécifiquement anti-HTLV-I ou HTLV-II, caractérisé en ce que le fragment d'ADN est choisi parmi un fragment Bam H1-Xho1 d'une longueur de ~ 397 paires de bases codant pour une séquence polypeptidique de 15,88 Kd et un fragment Xho1-Bam H1 de ~ 321 paires de bases codant pour une séquence polypeptidique de 12,84 Kd.

**15.** Procédé pour analyser en sérologie la présence dans des sérums humains d'anticorps anti-HTLV-I ou HTLV-II et anti-retrovirus humains apparentés, comprenant l'utilisation du produit de la revendication 14.

**16.** Procédé selon l'une des revendications 14 ou 15, caractérisé en ce qu'un fragment individuel de gène de l'enveloppe de HTLV-I est inséré dans le vecteur plasmide pJLA16 au moyen d'un segment de liaison polynucléotidique, ledit vecteur est introduit dans E. coli, et ledit E. coli est mis en culture à 32°C et induit à 42°C par une réaction de sensibilité à la température.

**17.** Procédé selon la revendication 16, caractérisé en ce que E. Coli est E. coli MZ1.

**18.** Fragment Xho1-Bam H1 de gène de l'enveloppe ayant ~ 321 paires de base codant pour un polypeptide qui contient des sites antigéniques présentant une réactivité vis-à-vis d'anticorps humains dirigés contre HTLV-I ou HTLV-II ou des rétrovirus humains apparentés.

**19.** Fragment Bam H1-Xho1 de gène de l'enveloppe de HTLV-I codant pour un polypeptide contenant des sites antigéniques présentant une réactivité vis-à-vis d'anticorps humains dirigés contre HTLV-I ou HTLV-II ou des rétrovirus humains apparentés.

**20.** Antigène de l'enveloppe produit dans E. coli portant le vecteur pJLA16 dans celui où a été inséré un fragment du gène de l'enveloppe selon l'une des revendications 18 ou 19 en quantité convenable pour détecter un anticorps dans des sérums humains spécifiquement dirigé contre HTLV-I.

**Patentansprüche**

**1.** Verfahren zur Herstellung von retroviralen HTLV-I-Hüllproteinen mit den Schritten:
(1) Isolieren des Hüllgens eines menschlichen lymphotropen Retrovirus HTLV-I,
(2) Spalten des Hüllgens zur Herstellung von Genfragmenten, die jeweils für spezifische Regionen der Hülle codieren,
(3) Einfügen eines in Schritt (2) gebildeten Fragments in einen Vektor,
(4) Transferieren des in Schritt (3) gebildeten Vektors in einen Prokaryont-Wirt, und Lysieren der Wirtszellen, und
(5) Isolieren des genannten HTLV-I-Retrovirus-Hüllproteins aus den lysierten Wirtszellen,

dadurch gekennzeichnet, daß das abgespaltene Fragment nach Schritt (2) mindestens ein Genfragment ist, das für ein Polypeptid codiert, das mit menschlichen Antikörpern gegen HTLV-I oder HTLV-II oder verwandte menschliche Retroviren reaktive antigene Stellen enthält, wobei das Fragment aus einem ~ 312 Basenpaar-Xho1-Bam H2-Hüllgenfragment und einem ~ 397 Basenpaar Bam H1-Xho1-Hüllgenfragment ausgewählt ist.

**2.** Verfahren nach Anspruch 1, dadurch gekennzeichnet, daß die Fragmente nach Schritt (3) in den Vektor so eingefügt werden, daß ein Teil des Lambda-cII-Gens stromauf des eingefügten Fragments liegt.

**3.** Verfahren nach Anspruch 1 oder Anspruch 2, dadurch gekennzeichnet, daß das Hüllgen in zwei Fragmente gespalten wird, von denen das eine für ein Glycoprotein codiert und das andere für ein Transmembran-Protein codiert.

**4.** Verfahren nach einem der Ansprüche 1 bis 3, dadurch gekennzeichnet, daß der Vektor nach Schritt (3) ein Plasmid ist.

5. Verfahren nach einem der vorhergehenden Ansprüche, dadurch gekennzeichnet, daß die Wirtszelle E. coli ist.

6. Verfahren nach Anspruch 5, dadurch gekennzeichnet, daß das E. coli das E. coli MZ1 ist, das einen mutanten C1-857 temperaturempfindlichen Repressor trägt.

7. Verfahren nach einem der vorhergehenden Ansprüche, dadurch gekennzeichnet, daß der Vektor nach Schritt (3) ein pJLA16-Plasmid ist.

8. Verfahren nach Anspruch 7, dadurch gekennzeichnet, daß der inserierte Vektor pKS400 ist, der unter ATCC Nr. 39903 hinterlegt ist.

9. Verfahren nach Anspruch 7, dadurch gekennzeichnet, daß der inserierte Vektor pKS300 ist, der unter ATCC Nr. 39902 hinterlegt ist.

10. Zusammensetzung, die Peptide aufweist, die nach dem Verfahren nach Anspruch 1 auf einer festen Unterlage oder einem festen Träger hergestellt sind.

11. Verfahren zum Nachweis von Antikörpern zu HTLV-I, wobei eine Zusammensetzung nach Anspruch 10 mit Seren in Berührung gebracht wird, in denen Antikörper zu HTLV-I vermutet werden.

12. Verfahren nach Anspruch 11, dadurch gekennzeichnet, daß die Peptide an einer festen Unterlage oder einem festen Träger befestigt sind.

13. Verfahren nach Anspruch 12, dadurch gekennzeichnet, daß die Peptide unter Verwendung eines ELISA-Tests untersucht werden.

14. Verfahren zum Exprimieren eines DNA-Fragments aus der Hüllcodierungsregion von HTLV-I in einem bakteriellen Vektor, wobei das durch das Fragment dargestellte Polypeptid zum Antikörpernachweis von spezifisch gegen HTLV-I oder HTLV-II gerichteten Antikörpern in menschlichen Seren verwendet werden kann, dadurch gekennzeichnet, daß das DNA-Fragment aus einem Bam H1-Xho1-Fragment mit einer Länge von ~ 397 Basenpaaren, das für eine 15,88 Kd-Polypeptidsequenz codiert, und einem Xho1-Bam H1-Fragment von ~ 321 Basenpaaren, das für eine 12:84 Kd-Polypeptidsequenz codiert, ausgewählt ist.

15. Verfahren zur serologischen Untersuchung menschlicher Seren auf gegen HTLV-I oder HTLV-II und verwandte menschliche Retroviren gerichtete Antikörper, bei welchem das Produkt nach Anspruch 14 verwendet wird.

16. Verfahren nach Anspruch 14 oder 15, dadurch gekennzeichnet, daß ein individuelles Hüllgenfragment von HTLV-I in den Plasmidvektor pJLA16 unter Verwendung eines Polynukleotid-Linkers inseriert wird, dieser Vektor in E. coli eingeführt wird und dieses E. coli bei 32˚C gezüchtet und bei 42˚C in einer temperaturempfindlichen Reaktion induziert wird.

17. Verfahren nach Anspruch 16, dadurch gekennzeichnet, daß das E. coli das E. coli MZ1 ist.

18. Das ~ 321 Basenpaar-Xho1-Bam H1-Hüllgenfragment von HTLV-I, das für ein Polypeptid codiert, das mit gegen HTLV-I oder HTLV-II oder verwandte menschliche Retroviren gerichteten menschlichen Antikörpern reaktive antigene Stellen enthält.

19. Das ~ 397 Basenpaar-Bam H1-Xho1-Hüllgenfragment von HTLV-I, das für ein Polypeptid codiert, das mit gegen HTLV-I oder HTLV-II oder verwandte menschliche Retroviren gerichteten menschlichen Antikörpern reaktive antigene Stellen enthält.

20. Hüllantigen, das in E. coli produziert ist, das den Vektor pJLA16 trägt, in welchen ein Hüllgenfragment nach Anspruch 18 oder 19 in einer zum Nachweis von spezifisch gegen HTLV-I gerichteten Antikörpern

EP 0 181 107 B1

in menschlichen Seren geeigneten Menge inseriert worden ist.

# FIG.1

# FIG.2

# FIG.3